# EUROPEAN PATENT APPLICATION

(11) **EP 4 335 492 A1**
(43) Date of publication of application: **13.03.2024**
(21) Application number: 23194411.7
(22) Date of filing: 31.08.2023
(51) Int. Cl.: A61N 1/375, A61N 1/05, A61N 1/372

(54) **BIOSTIMULATOR HAVING PIVOTABLE ELECTRODE**

(30) Priority: 07.09.2022 US 202263404449 P; 28.08.2023 US 202318239059
(71) Applicant: Pacesetter, Inc., Sylmar, CA 91342 (US)
(72) Inventor: CHANTASIRIVISAL, Steve, Sylmar, 91342 (US); VICTORINE, Keith, Sylmar, 91342 (US); MEDINA, Christian, Sylmar, 91342 (US); SOMOGYI, Zoltan, Sylmar, 91342 (US); KWON, David, Sylmar, 91342 (US); CHEN, Xiagqun Shawn, Sylmar, 91342 (US)
(74) Representative: Barker Brettell Sweden AB

(57) **Abstract**

A biostimulator (100) and a biostimulator system (200) for septal pacing, is described. The biostimulator (100) includes a joint (306) to allow a pacing electrode (106) to pivot relative to a housing (108). The housing (108) contains electrical circuitry that is electrically connected to the pacing electrode (106). The joint (306) allows the pacing electrode (106) to affix to target tissue of an interventricular septal wall (104) of a heart (102) when the housing (108) is pivoted toward an apex (105) of the heart (102).

## Description

### TECHNICAL FIELD

The present disclosure relates to biostimulators and related biostimulator systems. More specifically, the present disclosure relates to leadless biostimulators and related systems useful for septal pacing.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right ventricle and, for dual-chamber pacing, in a right atrium, by an anchor. A delivery system can be used to deliver the leadless cardiac pacemakers to the target anatomy.

Cardiac pacing of the His-bundle is clinically effective and advantageous by providing a narrow QRS affecting synchronous contraction of the ventricles. His-bundle pacing in or near a membranous septum of a heart, however, has some drawbacks. The procedure is often long in duration and requires significant fluoroscopic exposure. Furthermore, successful His-bundle pacing cannot always be achieved. Pacing thresholds are often high, sensing is challenging, and success rates can be low.

Pacing at the left bundle branch (LBB) is an alternative to His-bundle pacing. Pacing at the LBB involves pacing past the His-bundle toward the right ventricular apex. More particularly, a pacing site for LBB pacing is typically below the His-bundle, on the interventricular septal wall. To achieve optimal results, the pacing site for physiological pacing at the LBB can be high on the interventricular septal wall, in the region close to the tricuspid valve and pulmonary artery outflow track. Optimal pacing may require that a pacing electrode be inserted normal to the septal wall. Achieving such an insertion angle can require several implant attempts.

### SUMMARY

Existing leadless pacemakers may not fit, or may interfere with heart structures, when placed at the optimal pacing site for left bundle branch (LBB) pacing. Existing leadless pacemakers have bodies that are long and rigid and, when implanted at the interventricular septal wall, could extend into contact with and bruise the cardiac tissue of a ventricular free wall or interfere with the tricuspid valve. The long and rigid body of existing leadless pacemakers could also become tangled within chordae tendinae. Furthermore, a proximal end of the existing leadless pacemakers may flail within the heart chamber as the heart beats, causing cyclical contact with adjacent heart structures. Such contact could interfere with heart function. In addition to exceeding space constraints associated with LBB pacing, and interfering with heart function, the rigidity and lack of angular mobility of existing leadless pacemakers and pacemaker delivery systems can make it difficult to insert a pacing electrode of the leadless pacemakers normal to the septal wall. More particularly, existing systems do not have angular flexibility and are difficult to deploy several times to find an acceptable location for the pacing electrode. For the reasons above, there is a need for a leadless biostimulator that can be engaged to the interventricular septal wall to pace the LBB without interfering with adjacent structures of the heart. There is also a need for a leadless biostimulator that has angular flexibility to facilitate insertion of a pacing electrode normal to the septal wall, potentially after several deployment attempts.

The present invention is defined in the independent claim. Further embodiments of the invention are defined in the dependent claims.

A biostimulator is described. The biostimulator includes a pacing electrode electrically connected to pacing circuitry within a housing. The biostimulator includes a joint between the pacing electrode and the housing. The j oint allows the pacing electrode to pivot relative to the housing. Accordingly, the pacing electrode can engage tissue of a septal wall to pace an LBB and the housing can pivot in a direction of a ventricular apex to avoid contacting sensitive heart structures.

In an embodiment, the joint includes a spherical bearing. For example, a ball may be connected to the pacing electrode, and a header assembly having a socket can be connected to the housing. The ball may be located and movable within the socket. Accordingly, the spherical bearing can allow the pacing electrode to tilt and rotate relative to the housing.

In an embodiment, the biostimulator includes a torque element. The torque element can be connected at a distal element end to the pacing electrode, and at a proximal element end to the housing. The torque element can have a torsional stiffness that allows torque to be transmitted from the housing to the pacing electrode. For example, the pacing electrode can include a helical electrode, and torque can be transmitted through the housing and the torque element to the helical electrode to screw the pacing electrode into the target pacing site. Accordingly, the pacing electrode can tilt relative to the housing and my twist in unison with the housing.

In an embodiment, the joint includes a universal joint. A conductor can extend through the universal joint between the pacing circuitry and the pacing electrode. For example, the conductor can pass through a channel of a spider of the universal joint. The spider can include an annulus having a tapered surface extending distally from the channel, and several pins radiating outward from the annulus. The spider can interconnect a driving yoke and a driven yoke that pivot relative to each other. Accordingly, the universal j oint can transmit torque while pivoted to a driving angle such that, when torquing the housing to screw the pacing electrode into the interventricular septal wall, the electrode axis and the housing axis remain at the driving angle.

A biostimulator system is described. In an embodiment, the biostimulator system includes a biostimulator transport system. The biostimulator can be mounted on the biostimulator transport system to carry the biostimulator to or from the target anatomy.

The above summary does not include an exhaustive list of all aspects of the present invention. It is contemplated that the invention includes all systems that can be practiced from all suitable combinations of the various aspects summarized above, as well as those disclosed in the Detailed Description below and particularly pointed out in the claims filed with the application. Such combinations have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings.
FIG. 1 is a diagrammatic cross section of a patient heart illustrating an example implantation of a biostimulator in a target anatomy, in accordance with an embodiment.
FIG. 2 is a perspective view of a biostimulator system, in accordance with an embodiment.
FIG. 3 is a perspective view of a biostimulator having a pivotable electrode, in accordance with an embodiment.
FIG. 4 is a side view of a biostimulator having a pivotable electrode, in accordance with an embodiment.
FIG. 5 is an end view of a biostimulator having a pivotable electrode, in accordance with an embodiment.
FIG. 6 is a sectional view of a biostimulator having a pivotable electrode, in accordance with an embodiment.
FIG. 7 is a sectional view of a biostimulator having a pivotable electrode, in accordance with an embodiment.
FIG. 8 is a sectional view of a biostimulator having a pivotable electrode, in accordance with an embodiment.
FIG. 9 is a perspective view of a biostimulator having a movable fixation helix in a retracted state, in accordance with an embodiment.
FIG. 10 is a perspective view of a biostimulator having a movable fixation helix in an extended state, in accordance with an embodiment.
FIG. 11 is a side view of a biostimulator having a movable fixation helix in a retracted state, in accordance with an embodiment.
FIG. 12 is a side view of a biostimulator having a movable fixation helix in an extended state, in accordance with an embodiment.
FIG. 13 is a flowchart of a method of implanting a biostimulator for septal pacing.
FIG. 14 is a perspective view of a biostimulator having a pivotable electrode, in accordance with an embodiment.
FIG. 15 is a perspective view of a biostimulator having a pivotable electrode, in accordance with an embodiment.
FIG. 16 is a perspective view of a joint of a biostimulator, in accordance with an embodiment.
FIG. 17 is a perspective view of a spider of a joint of a biostimulator, in accordance with an embodiment.
FIGS. 18-20 are side views of a biostimulator having a pivotable electrode in various pivoted positions, in accordance with an embodiment.
FIGS. 21-22 are perspective views of a biostimulator having a pivotable electrode in various torqued positions, in accordance with an embodiment.

### DETAILED DESCRIPTION

Embodiments describe a biostimulator and a biostimulator system for septal pacing. The biostimulator may, however, be used in other applications, such as deep brain stimulation. Thus, reference to the biostimulator as being a cardiac pacemaker for septal pacing is not limiting.

In various embodiments, description is made with reference to the figures. However, certain embodiments may be practiced without one or more of these specific details, or in combination with other known configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a biostimulator. Similarly, "proximal" may indicate a second direction opposite to the first direction. Such terms are provided to establish relative frames of reference, however, and are not intended to limit the use or orientation of a biostimulator to a specific configuration described in the various embodiments below.

In an aspect, a biostimulator includes a j oint to allow an electrode axis of a pacing electrode to be directed differently than a housing axis of a housing. For example, the pacing electrode can be a helical electrode that affixes to an interventricular septal wall and the electrode axis can extend normal to the septal wall, while the housing can be pivotably directed toward a ventricular apex along the septal wall. The housing can be located near the apex and the housing axis can be normal to an apex wall. Accordingly, when the helical electrode is anchored in a septal wall of a heart, the housing can be located in the ventricular apex without interfering with a heart valve or an outer heart wall opposite to the septal wall. The biostimulator therefore fits well within the limited space of the target heart chamber, and provides long-term implant stability. A biostimulator system is described that can transport the biostimulator to or from a pacing site at the septal wall.

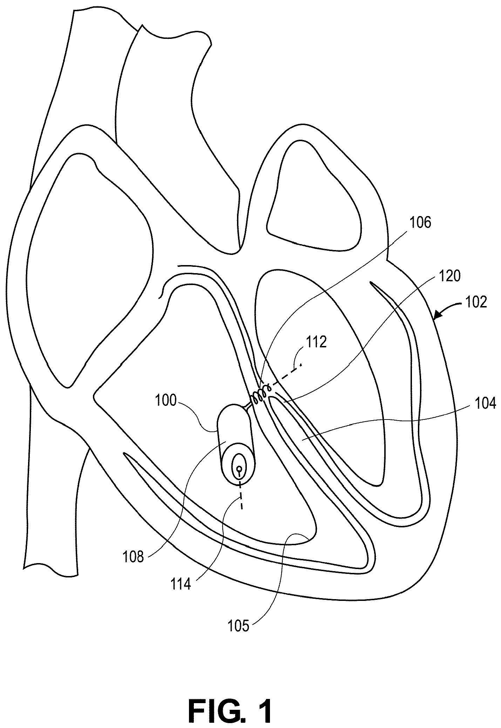

Referring to FIG. 1, a diagrammatic cross section of a patient heart illustrating an example implantation of a biostimulator in a target anatomy is shown in accordance with an embodiment. A leadless biostimulator system, e.g., a cardiac pacing system, includes one or more biostimulators 100. The biostimulators 100 can be implanted in a patient heart 102, and can be leadless (and thus, may be leadless cardiac pacemakers). Each biostimulator 100 can be placed in a cardiac chamber, such as a right atrium and/or right ventricle of the heart 102, or attached to an inside or outside of the cardiac chamber. For example, the biostimulator 100 can be attached to one or more of an interventricular septal wall 104 or a ventricular apex 105 of the heart 102. More particularly, the biostimulator 100 can be delivered to an interventricular septum, and one or more elements, such as a pacing electrode 106, can pierce the interventricular septal wall 104 of the septum to engage and anchor the biostimulator 100 to the tissue. The biostimulator 100 can have a housing 108, which can be directed toward the ventricular apex 105, or otherwise pivoted relative to the pacing electrode 106, as described below.

The pacing electrode 106 can have an electrode axis 112, which extends centrally through the pacing electrode and is directed toward, e.g., normal to, the septal wall when the pacing electrode 106 is affixed to the septal wall. Similarly, the housing 108 can have a housing axis 114, which extends centrally through the housing and is directed away from, e.g., oblique to, the septal wall 104 when the pacing electrode 106 is affixed to the septal wall 104. For example, the housing axis 114 can be directed toward an apex wall of the ventricular apex 105 and the housing 108 may be located therein.

When the pacing electrode 106 is affixed to the interventricular septal wall 104, and the housing 108 is located at the ventricular apex 105, the electrode axis 112 can extend in a different direction than the housing axis 114. For example, the electrode axis 112 can extend in a direction that is transverse or oblique to a direction of the housing axis 114. The non-coaxial and/or non-parallel relationship of the electrode axis 112 and the housing axis 114 may be provided by a joint of the biostimulator 100, as described below.

When the biostimulator 100 is delivered to and screwed into the septum of the heart 102, the pacing electrode 106 may be positioned for deep septal pacing at a target bundle branch 120 in the septum. For example, an active electrode of the pacing element can be positioned at the left bundle branch 120 in the septum. The biostimulator 100 may deliver pacing impulses through the pacing electrode 106 to the bundle branch(es). Accordingly, the pacing electrode 106 can be located to effectively probe and pace the left bundle branch 120, while the housing 108 can be placed in a safe and non-obstructive location within the heart chamber.

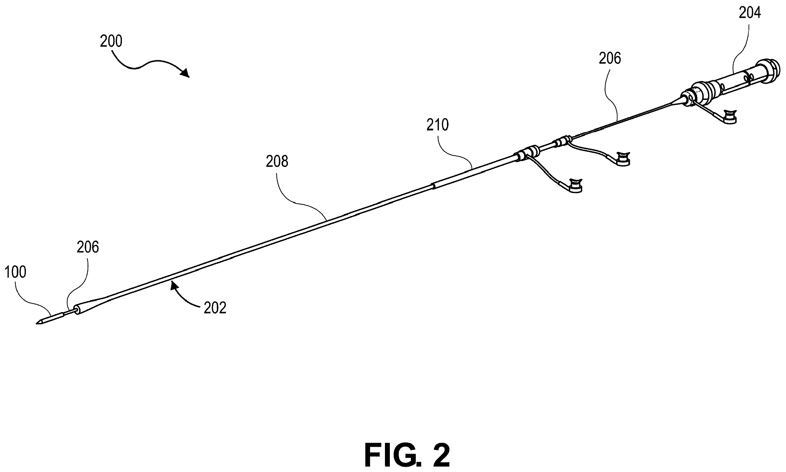

Referring to FIG. 2, a perspective view of a biostimulator system is shown in accordance with an embodiment. Leadless pacemakers or other leadless biostimulators 100 can be delivered to or retrieved from a patient using delivery or retrieval systems. The leadless biostimulator system can include delivery or retrieval systems, which may be catheter-based systems used to carry a leadless biostimulator 100 intravenously to or from a patient anatomy. The delivery or retrieval systems may be referred to collectively as transport systems, or biostimulator transport systems.

A biostimulator system 200 can include a biostimulator transport system 202. The biostimulator 100 can be attached, connected to, or otherwise mounted on the biostimulator transport system 202. For example, the biostimulator 100 can be mounted on a distal end of a catheter of the biostimulator transport system 202. The biostimulator 100 is thereby advanced intravenously into or out of the heart 102.

The biostimulator transport system 202 can include a handle 204 to control movement and operations of the transport system from outside of a patient anatomy. One or more elongated members extend distally from the handle 204. For example, a support member 206 can extend distally from the handle 204. The support member 206 can extend to a distal end of the transport system 202. In an embodiment, the biostimulator 100 is mounted on the biostimulator transport system 202, e.g., at a distal end of the support member 206.

The biostimulator transport system 202 can include a protective sleeve 208 to cover the biostimulator 100 during delivery and implantation. The protective sleeve 208 can extend over, and be longitudinally movable relative to, the support member 206. The biostimulator transport system 202 may also include an introducer sheath 210 that can extend over, and be longitudinally movable relative to, the protective sleeve 208. The introducer sheath 210 can cover a distal end of the protective sleeve 208, the support member 206, and the biostimulator 100 as those components are passed through an access device into the patient anatomy.

Several components of the biostimulator transport system 202 are described above by way of example. It will be appreciated, however, that the biostimulator transport system 202 may be configured to include additional or alternate components. More particularly, the biostimulator transport system 202 may be configured to deliver and/or retrieve the biostimulator 100 to or from the target anatomy. Delivery and/or retrieval of the biostimulator 100 can include retaining the biostimulator 100 during transport to the target anatomy and rotation of the biostimulator 100 during implantation of the biostimulator at the target anatomy. Accordingly, the biostimulator transport system 202 can incorporate features to retain and rotate the biostimulator 100.

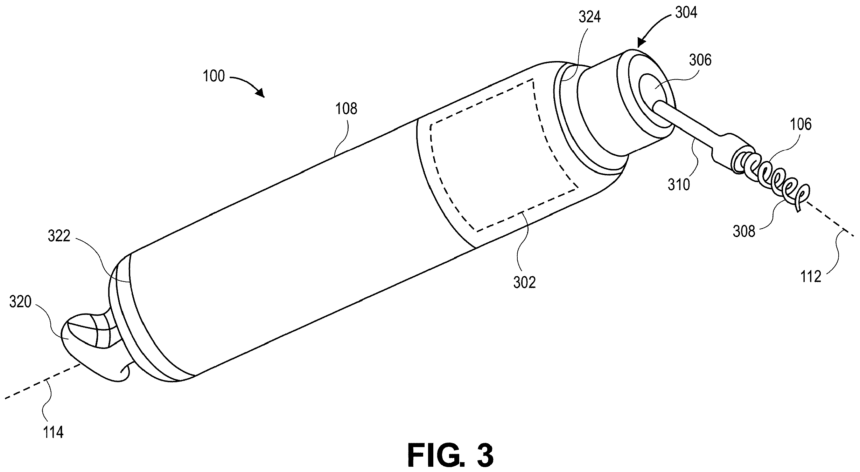

Referring to FIG. 3, a perspective view of a biostimulator having an articulable extension is shown in accordance with an embodiment. The biostimulator 100 can be a leadless cardiac pacemaker that can perform cardiac pacing and that has many of the advantages of conventional cardiac pacemakers while extending performance, functionality, and operating characteristics. In a particular embodiment, the biostimulator 100 can use two or more electrodes located on or within a housing 108 of the biostimulator 100 for pacing the cardiac chamber upon receiving a triggering signal from at least one other device within the body. The two or more electrodes of the biostimulator 100 can include the pacing electrode 106 that acts as an active electrode and/or a portion of the housing 108 that acts as an active electrode. The electrodes can deliver pacing pulses to bundle branches 120 within the septum of the heart 102 to perform deep septal pacing, and optionally, can sense electrical activity from the muscle. The electrodes may also communicate bidirectionally with at least one other device within or outside the body.

The housing 108 has a longitudinal axis, e.g., the housing axis 114. The housing 108 can contain a primary battery to provide power for pacing, sensing, and communication, which may include, for example, bidirectional communication. The housing 108 can optionally contain an electronics compartment 302 (shown by hidden lines) to hold circuitry adapted for different functionality. For example, the electronics compartment 302 can contain pacing circuitry for sensing cardiac activity from the electrodes, for receiving information from at least one other device via the electrodes, for generating pacing pulses for delivery to tissue via the pacing electrode 106, or other circuitry. Accordingly, the pacing circuitry can be electrically connected to the pacing electrode 106. The electronics compartment 302 may contain circuits for transmitting information to at least one other device via the electrodes and can optionally contain circuits for monitoring device health. The circuitry of the biostimulator 100 can control these operations in a predetermined manner. In some implementations of a cardiac pacing system, cardiac pacing is provided without a pulse generator located in the pectoral region or abdomen, without an electrode-lead separate from the pulse generator, without a communication coil or antenna, and without an additional requirement of battery power for transmitted communication.

In an embodiment, the biostimulator 100 includes a pivotable electrode. The biostimulator 100 may include a header assembly 304 mounted on the housing 108. The header assembly 304 can include a joint 306. The joint 306 can be between the pacing electrode 106 and the housing 108. More particularly, the joint 306 can have a portion coupled to the pacing electrode 106, and another portion coupled to the housing 108. The portions can swivel or pivot relative to each other. Accordingly, the pacing electrode 106 is pivotable relative to the housing 108. The joint 306 allows the pacing electrode 106 to be located at the pacing site at a location on the septal wall 104 nearer to the heart valve than the housing 108 while the housing is located, e.g., at the ventricular apex 105 for implant stability.

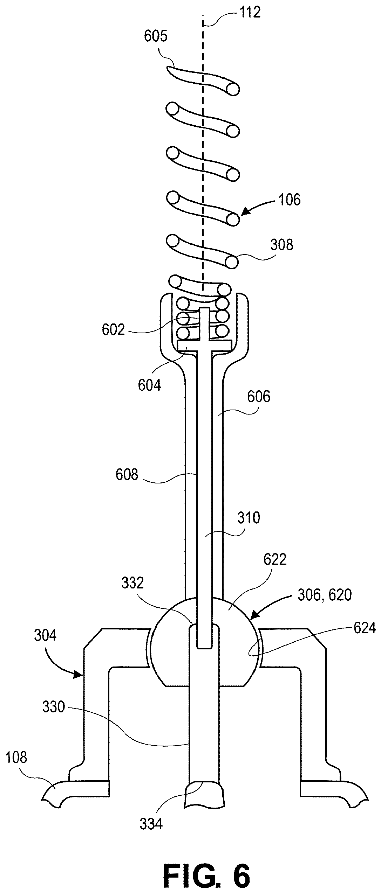

The header assembly 304 of the biostimulator 100 can include components to fix the biostimulator 100 to an intracardial implant site, e.g., at the septal wall. More particularly, the header assembly 304 may include one or more actively engaging mechanisms or fixation mechanisms, such as a helical electrode 308. In an embodiment, the pacing electrode 106 includes the helical electrode 308. The helical electrode 308 can include a screw or helical member that screws into the myocardium. The helical electrode 308 can be connected to an electrode shaft 310 of the pacing electrode 106. More particularly, the electrode shaft 310 can extend distally from the joint 306, and the helical electrode 308 can be mounted on a distal shaft end (FIG. 6) of the electrode shaft 310. Accordingly, when the helical electrode 308 is implanted into a target tissue, the electrode shaft 310 can tether the housing 108 to the helical electrode 308 through the joint 306.

In an embodiment, the biostimulator 100 includes an attachment feature 320. The attachment feature 320 can be mounted on a proximal housing end 322 of the housing 108. More particularly, the attachment feature 320 can be mounted on an opposite end of the housing 108 from the pacing electrode 106, which can be a component of the header assembly 304 coupled to the distal housing end 324 of the housing 108. The attachment feature 320 can facilitate precise delivery or retrieval of the biostimulator 100. For example, the attachment feature 320 can be formed from a rigid material to allow a delivery or retrieval system to engage the attachment feature 320 and transmit torque through the housing 108 and electrode shaft 310 to screw the pacing electrode 106 into the target tissue.

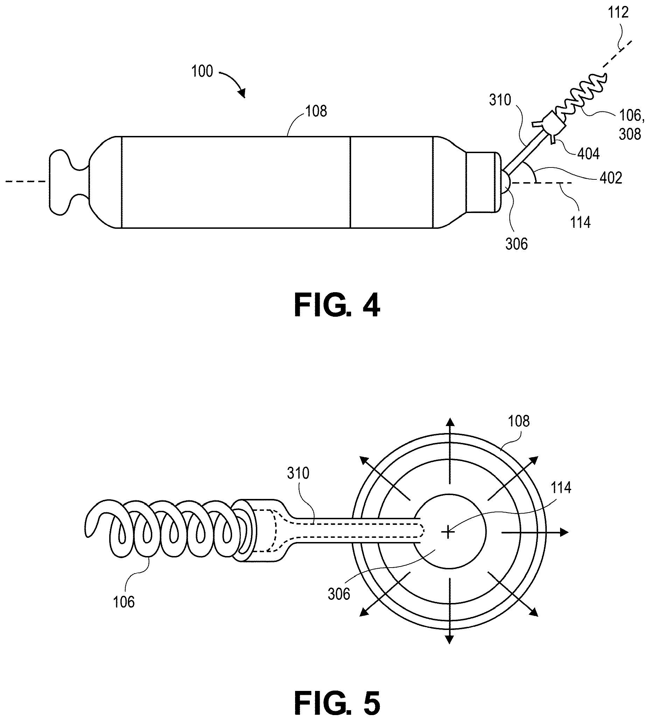

Referring to FIG. 4, a side view of a biostimulator is shown in accordance with an embodiment. The joint 306 of the biostimulator 100 allows the pacing electrode 106 to pivot in a variety of different directions relative to the housing 108. The housing 108 can be pivoted relative to the pacing electrode 106 to avoid a tricuspid valve and/or contact with a ventricular free wall during contraction. The joint 306 can have a range of motion that allows electrode axis 112 to move into a non-coaxial orientation relative to the housing axis 114. For example, in a side view, the range of motion can have a lateral pivot angle 402 in a range of 0° to 60°, e.g., up to 45°. The lateral pivot angle 402 can be a half-angle, measured within a longitudinal plane containing the housing axis 114 and the electrode axis 112, between the electrode axis 112 and the housing axis 114. A full-angle may be twice the lateral pivot angle 402, and may be symmetric about the housing axis 114 within the longitudinal plane. Accordingly, the lateral pivot angle 402 provides for the electrode to be in a straight configuration, in which the electrode axis 112 is parallel to the housing axis 114, or in a pivoted configuration, in which the electrode axis 112 is oblique to the housing axis 114, as shown.

As described above, the helical electrode 308 of the pacing electrode 106 can be used to pace tissue, as well as to affix the biostimulator 100 to the target tissue. In an embodiment, the pacing electrode 106 includes one or more backstop elements 404 configured to retain the pacing electrode 106 in the target tissue when the helical electrode 308 is affixed to the target tissue. The backstop elements 404 can include sutures directed radially outward from the electrode axis 112. The sutures, which may be short segments of suture that form teeth to grip the target tissue, can extend radially outward and/or in an oblique direction relative to the electrode axis 112. For example, the backstop elements 404 can be backward facing sutures, directed in a rearward and laterally outward direction, to grip tissue. More particularly, when the helical electrode 308 is implanted within the target tissue, the backstop elements 404 may be embedded within tissue such that any backward movement of the housing 108 will press the proximal ends of the backstop elements 404 against the tissue within which they are embedded. Backstop elements 404 will therefore engage the tissue and resist backout of the pacing electrode 106.

Referring to FIG. 5, an end view of a biostimulator is shown in accordance with an embodiment. The joint 306 of the biostimulator 100 can allow the pacing electrode 106 to rotate or spin about the housing axis 114. More particularly, when viewed in a direction of the housing axis 114, with the electrode shaft 310 deflected to the lateral pivot angle 402, the electrode shaft 310 can rotate about the housing axis 114 in either a clockwise or counterclockwise direction. In an embodiment, the pacing electrode 106 can pivot in any direction. More particularly, movement of the pacing electrode 106 about the housing axis 114 may be provided over a full 360° range. Alternatively, movement about the housing axis 114 can be restricted to a partial rotational range, e.g., in a range of 1° to 270° about the housing axis 114. By permitting movement of the pacing electrode 106 in both the lateral pivot angle direction (FIG. 4) and the rotational direction about the housing axis 114 (FIG. 5), the pacing electrode 106 has sufficient degrees of freedom to allow the housing 108 to pivot freely relative to the pacing electrode 106. Accordingly, the housing 108 can be moved toward the ventricular apex 105 and out of the way of sensitive heart structures when the pacing electrode 106 is implanted at the left bundle branch 120.

Referring to FIG. 6, a sectional view of a biostimulator is shown in accordance with an embodiment. The cross-sectional illustration shows the structure and interconnections between components of the pacing electrode 106 and the joint 306. As described above, the electrode shaft 310 can have a distal shaft end 602, and the helical electrode 308 may be mounted on the distal shaft end 602. For example, the electrode shaft 310 can have a central pin, and a pin flange 604 located in a proximal direction from the distal shaft end 602. The pin flange 604 can be a transverse, circular ledge, for example. A proximal end of the helical electrode 308 can press against and/or be bonded to the pin flange 604. The pin can extend into a central channel of the helical electrode 308. Accordingly, the helical electrode 308 can be radially centered on the electrode shaft 310, and may have a predefined longitudinal location relative to the electrode shaft 310. A combined distance from the joint 306 to a distal electrode end 605 can be in a range of 0.5 to 1.5 cm, e.g., 1 cm, to allow the pacing electrode 106 to burrow into the septal wall 104 and engage the left bundle branch (LBB).

In an embodiment, a masking element 606 covers an outer surface 608 of the electrode shaft 310. The masking element 606 can include a sleeve, a film, a coating, or another covering that insulates the electrode shaft 310 from the surrounding environment. For example, as shown, the masking element 606 may be a thin-walled tubing that is placed around, and conforms to the outer surface 608 of the electrode shaft 310. The masking element 606 can extend from the joint 306, at a proximal end, onto and over at least a portion of the helical electrode 308.

Covering a portion of the helical electrode 308 can influence an impedance of the electrical pathway from the biostimulator 100 to the target tissue. For example, exposing more of the helical electrode 308 to the surrounding environment can decrease impedance, and vice versa. In an embodiment, the masking element 606 surrounds and covers all of the helical electrode 308 except for a most distal 1-2 turns of the helical electrode 308. More particularly, the most distal 1-2 turns of the helical electrode 308 can be exposed to the surrounding environment. Accordingly, the masking element 606 can isolate a portion of the pacing electrode 106 and control impedance of the pacing electrode 106 to control the delivery of pacing impulses.

In an embodiment, the joint 306, which can interconnect the pacing electrode 106 to the housing 108, can include a spherical bearing 620. For example, the header assembly 304 can include a ball 622 and a socket 624. More particularly, the ball 622 can be located or disposed in the socket 624 to form a spherical joint between the electrode shaft 310 and the header assembly 304.

The joint 306 can allow free rotation between the electrode shaft 310 and the header assembly 304. For example, the ball 622 can spin freely within the socket 624 absent a constraining force. In an embodiment, however, the biostimulator 100 includes a constraint to limit relative movement of components of the joint 306, e.g., relative movement between the ball 622 and the socket 624. The biostimulator 100 can include a torque element 330 having a distal element end 332 connected to the pacing electrode 106, and a proximal element end 334 connected to a fixed location within the biostimulator 100. For example, the proximal element end 334 can be connected, physically and/or electrically, to the pacing circuitry within the electronics compartment 302.

The torque element 330 can have a torsional stiffness that limits rotation of the ball 622 about the electrode axis 112 or the housing axis 114. In an embodiment, the torque element 330 includes a tubular braid. Alternatively, the torque element 330 includes a tubular coil. The tubular element, whether coiled, woven, or otherwise formed, can be formed from one or more wires that are flexible and conductive. The individual wires may have round, rectangular, oval, or otherwise shaped cross-sectional areas. For example, a tubular braid can be formed from woven, round MP35N wires that are electrically conductive. Accordingly, the tubular structure of the torque element 330 can have an inherent torsional stiffness between the distal element end 332 and the proximal element end 334, and may also conduct electrical signals between the ends. Thus, the torque element 330 can transmit torque from the proximal element end 334 to the distal element end 332 during implantation, and can conduct electrical signals from the pacing circuitry to the electrode shaft 310 during tissue pacing. As an alternative to a coiled or braided torque element 330, the torque element 330 may include a flexible wire extending from the proximal element end 334 to the distal element end 332. The wire can include a thin, metallic wire having sufficient flexibility and torsional stiffness to allow the pacing electrode 106 to pivot and be screwed into the target tissue.

In addition to a wire coil, braid, or other structure, the torque element 330 can include supportive structures. For example, a thin-walled tubular sheathing, such as heat shrink, may be placed over a wire coil to prevent the coil from expanding in diameter when the coil is twisted, e.g., when torque is applied to the torque element 330. In an embodiment, the torque element 330 includes a single, metallic core wire to conduct electrical pulses, and a polymeric coil or braid surrounds the wire to provide additional mechanical strength to the component.

Torque can be transmitted to the torque element 330 during implantation through the attachment feature. More particularly, a transport system can twist the attachment feature, which transmits torque to the housing 108 and other components of the biostimulator 100 fixedly attached to the housing 108, such as the pacing circuitry, the header assembly 304, etc. The fixedly attached component of the biostimulator 100 may be connected to the proximal element end 334, and thus, torque can be transmitted to the torque element 330. When the helical electrode 308 is engaged with target tissue, and the torque transmitted through the torque element 330 exceeds the resistance torque of the tissue, the torque element 330 will transmit torque to the pacing electrode 106 to cause the helical electrode 308 to screw into the target tissue. For example, it has been found that the torque required to screw the helical electrode 308 into heart tissue is in a range of 0.2 to 0.9 in-oz. Accordingly, the torsional stiffness of the torque element 330 can be sufficient to achieve a required torque of 0.2 to 2.5 in-oz, e.g., 0.7 in-oz.

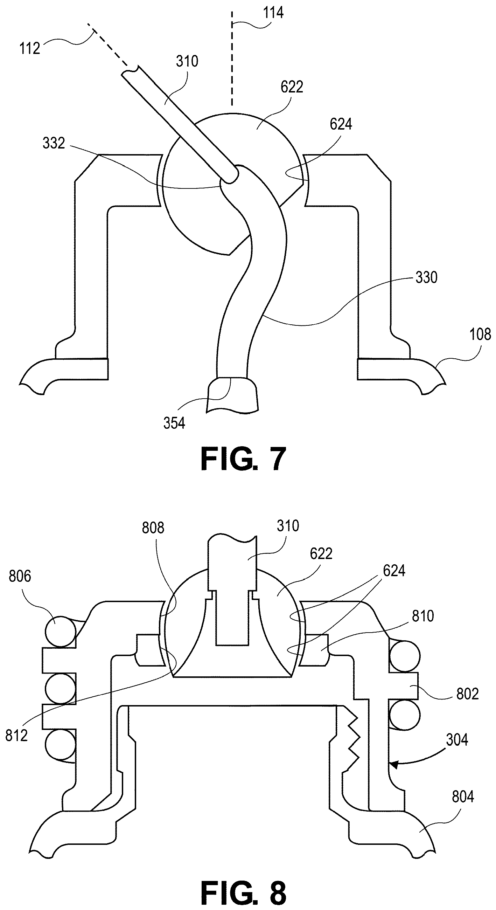

Referring to FIG. 7, a sectional view of a biostimulator is shown in accordance with an embodiment. In addition to having a torsional stiffness that transmits the required torque to the pacing electrode 106, the torque element 330 can have sufficient flexibility to allow the pacing electrode 106 to pivot relative to the housing 108. For example, as shown, when the electrode shaft 310 is tilted relative to the housing axis 114, the torque element 330 can bend to accommodate the tilting. More particularly, the torque element 330 can deform from a straightened configuration to a curved, e.g., S-curved, configuration to facilitate pivoting of the housing 108 relative to the pacing electrode 106 within the heart chamber.

Referring to FIG. 8, a sectional view of a biostimulator is shown in accordance with an embodiment. The joint 306 can include a socket 624 formed by several components of the header assembly 304. In an embodiment, the header assembly 304 includes a helix mount 802 mounted on a flange 804. The flange 804 can be a component of the header assembly 304 that connects to, e.g., is welded to, the housing 108. The flange 804 can hold an electrical feedthrough (not shown) that conducts electrical signals from the pacing circuitry within the housing 108 to the pacing electrode 106. More particularly, as shown in FIG. 7, the torque element 330 may connect to, and carry signals from, the electrical feedthrough to a more distal component.

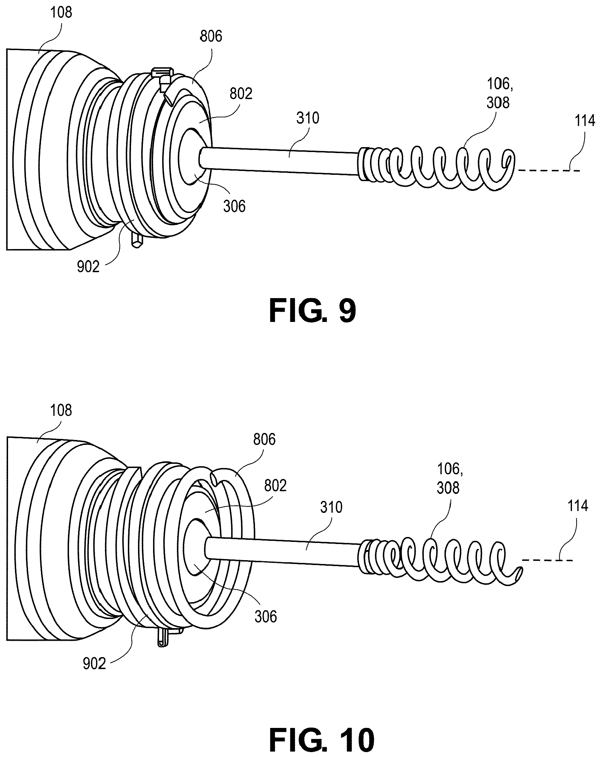

The helix mount 802 can be a component that receives a fixation helix 806. The fixation helix 806 is described further with respect to FIGS. 9-12, and may be a component used to secure the housing 108 to target tissue during implantation. The helix mount 802 can be screwed onto, or otherwise mounted on, the flange 804. In an embodiment, the helix mount 802 includes a first socket portion 808 that forms a portion of the socket 624. More particularly, the first socket portion 808 can include a concave surface surrounding a hole passing axially through an end face of the helix mount 802. The concave surface can be concave in a proximal direction. More particularly, the first socket portion 808 can have a radius of curvature that matches a radius of the ball 622. Accordingly, the first socket portion 808 can conform to the ball 622 such that, when the ball 622 is inserted into the first socket portion 808 proximal to the end face, the end face retains but allows free motion of the ball 622 within the first socket portion 808.

To secure the ball 622 within the socket 624, e.g., in a longitudinal direction, the header assembly 304 can include a backplate 810 that has a second socket portion 812. The second socket portion 812 forms a portion of the socket 624. More particularly, the second socket portion 812 can include a concave surface that combines with the concave surface of the first socket portion 808 to form the socket 624. The concave surface of the second socket portion 812 may be concave in a distal direction. Accordingly, when combined with the proximally concave surface of the first socket portion 808, a spherical contour can be formed between the combined helix mount 802 and backplate 810. In an embodiment, the spherical contour of the combined helix mount 802 and backplate 810 can conform to the spherical surface of the ball 622. Accordingly, the ball 622 can be retained in a longitudinal direction within the socket 624. The ball 622, although fixed in the longitudinal direction, may be freely movable within the socket 624 to provide the spherical bearing 620 that allows the pacing electrode 106 to pivot relative to the housing 108.

The backplate 810 can be mounted on the helix mount 802 to form the socket 624. For example, the backplate 810 can be attached to a proximal-facing surface of the helix mount 802 to create the socket 624 that retains the ball 622. In an embodiment, the backplate 810 is thermally or adhesively bonded to the helix mount 802. For example, the helix mount 802 and the backplate 810 may be formed from a same material, such as polyether ether ketone (PEEK), and thus, the backplate 810 can be ultrasonically welded to the helix mount 802. The components may also be dissimilar materials, and may be bonded using an adhesive.

Referring to FIG. 9, a perspective view of a biostimulator having a movable fixation helix 806 in a retracted state is shown in accordance with an embodiment. When the pacing electrode 106 is implanted within the septal wall 104, and the housing 108 is pivoted toward the ventricular apex 105, there may be a need to secure the housing 108 relative to the septal wall 104 to reduce a likelihood that the housing 108 will flail within the heart chamber. Accordingly, the biostimulator 100 can include a fixation helix 806 to secure the housing 108 to tissue within the heart chamber. The fixation helix 806 can be coupled to the housing 108, and thus, when the fixation helix 806 engages tissue, the housing 108 can be secured relative to the tissue. Accordingly, the housing 108 can be stabilized and, additionally, stress on the pacing electrode 106 can be reduced by avoiding a flailing housing pulling on the helical electrode 308.

As described above, the helix mount 802 may be mounted on, and fixed to, the housing 108. Similarly, the fixation helix 806 may be mounted on the helix mount 802. Rather than being fixed relative to the helix mount 802, however, in an embodiment the fixation helix 806 is movable relative to the helix mount 802. More particularly, the fixation helix 806 may be advanced or retracted, in a direction of the housing axis 114, relative to the helix mount 802. Accordingly, the fixation helix 806 may be advanced or retracted relative to the housing 108.

The fixation helix 806 can be threaded onto a helical thread of the helix mount 802. More particularly, the helix mount 802 can include a helix flange 902, which can be a square thread extending around, e.g., helically about, an outer surface 608 of the helix mount 802, about the housing axis 114. The fixation helix 806 can be screwed onto the helix flange 902. When the fixation helix 806 is rotated in a first rotational direction, e.g., clockwise, a distal tip of the fixation helix 806 can advance in a first longitudinal direction, e.g., forward. By contrast, when the fixation helix 806 is rotated in a second rotational direction, e.g., counter-clockwise, the distal tip of the fixation helix 806 can retract in a second longitudinal direction, e.g., backward.

The fixation helix 806 is shown in the retracted state in FIG. 9. In the retracted state, the distal tip of the fixation helix 806 can be flush with, or in a proximal direction from, the end face of the helix mount 802. Accordingly, when the end face contacts tissue, the retracted fixation helix 806 may be proximal to the tissue and therefore may not engage the tissue. This may be a preferred position when the pacing electrode 106 is being screwed into the target tissue.

Referring to FIG. 10, a perspective view of a biostimulator having a movable fixation helix 806 in an extended state is shown in accordance with an embodiment. After the pacing electrode 106 is screwed into the target tissue and the housing 108 is tilted to a stable location, an operator may want to secure the fixation helix 806 to tissue. The fixation helix 806 can be rotated relative to the helix mount 802 to advance the distal tip of the fixation helix. More particularly, the fixation helix 806 can be screwed forward to advance the distal tip beyond the end face of the helix mount 802. Accordingly, the distal tip can puncture tissue and the fixation helix 806 may be driven forward into the tissue. When the housing 108 is pivoted parallel to the septal wall 104, the fixation helix 806 can side stitch the tissue. The housing 108 may thereby be secured to the septal wall 104, and side loading of the pacing electrode 106 that could cause stress to the septal wall 104 may be reduced.

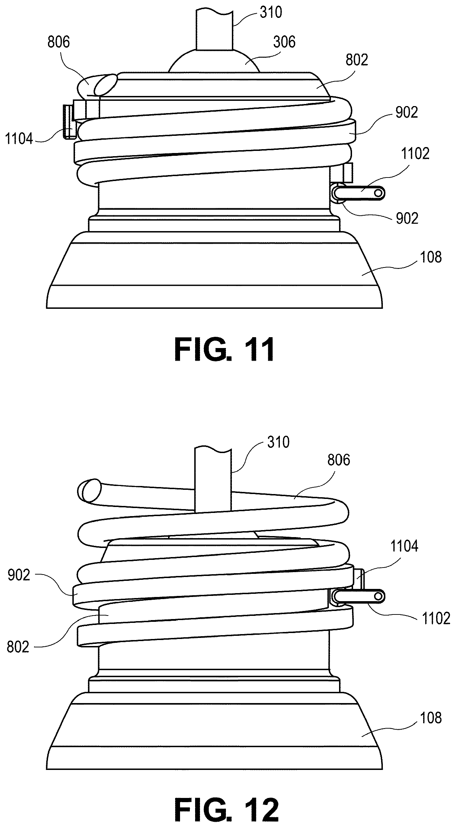

Referring to FIG. 11, a side view of a biostimulator having a movable fixation helix in a retracted state is shown in accordance with an embodiment. In an embodiment, the biostimulator 100 includes a mechanism to facilitate rotation of the fixation helix 806 relative to the helix mount 802. For example, the fixation helix 806 can include a tab 1102 extending radially outward, relative to the housing axis 114. The tab 1102 can be a prong, nub, ridge, etc. that extends outward to allow a corresponding mechanism of the transport system to engage the fixation helix 806. More particularly, the transport system mechanism can contact, grip, or otherwise engage the tab 1102 to apply a rotational load to the fixation helix 806 through the tab 1102. In the illustrated embodiment, the tab 1102 can be pushed forward (into the page, to cause the fixation helix 806 to rotate clockwise about the helix mount 802 and to advance the distal tip of the fixation helix 806.

Also shown in FIG. 11 is a stop 1104 extending from the helix flange 902. The stop 1104 can be a prong or protrusion positioned to engage and resist movement of the tab 1102 when the fixation helix 806 reaches a predetermined position within the helical groove of the helix mount 802.

Referring to FIG. 12, a side view of a biostimulator having a movable fixation helix in an extended state is shown in accordance with an embodiment. In the extended state, the fixation helix 806 can be at the predetermined position at which the tab 1102 of the fixation helix 806 contacts the stop 1104 of the helix mount 802. The stop 1104 can extend into the path of the tab 1102 such that, when the tab 1102 is advanced, the tab 1102 contacts the stop 1104 and further advancement of the fixation helix 806 is impeded. As shown, in the extended state, the distal tip of the fixation helix 806 can be distal to the end face of the helix mount 802. Accordingly, the fixation helix 806 can be engaged within tissue. The fixation helix 806 is retained within the helical groove of the helix mount 802 by the stop 1104, which avoids the fixation helix 806 being advanced off of the helix mount 802.

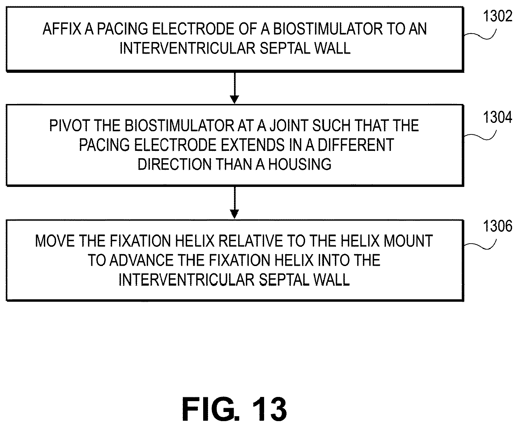

Referring to FIG. 13, a flowchart of a method of implanting a biostimulator for septal pacing is shown. During the implantation procedure, the biostimulator transport system 202 can carry the biostimulator 100 into the target heart chamber. When implantation is to be within the right ventricle, the biostimulator transport system 202 can be tracked through the inferior vena cava into the right atrium and across the tricuspid valve into the right ventricle. The distal end of the transport system can be steered toward a desired location of the septal wall 104. For example, the target area may be in an upper region of the interventricular septal wall 104.

At operation 1302, the pacing electrode 106 may be affixed to the interventricular septum. When a distal end of the biostimulator 100 is in contact with the septal wall 104, torque can be transferred from the biostimulator transport system 202 to the biostimulator 100, e.g., via the attachment feature. The torque can be applied to the attachment feature or the housing 108, and transmitted through the torque element 330 to the pacing electrode 106.

Rotation of the biostimulator 100 can drive the helical electrode 308 of the pacing electrode 106 into the septal tissue. The pacing electrode 106 can be screwed into the tissue to a desired depth by rotating the helices of the helical electrode 308 into the target tissue. The pacing electrode 106 may engage the tissue at a depth that allows effective pacing of the target bundle branch.

At operation 1304, the biostimulator 100 may be pivoted at the joint 306. For example, the biostimulator transport system 202 can be placed in a tether mode that allows the attachment feature to interconnect to the elongated member by flexible cables, without requiring the biostimulator 100 to be directly engaged to the protective sheath or the elongated member. In the tether mode, the housing 108 of the biostimulator 100 can be deflected downward toward the ventricular apex 105. More particularly, the electrode axis 112 of the pacing electrode 106 can extend in a first direction, and the housing 108 can be pivoted such that the housing axis 114 extends in a different direction than the electrode axis 112. Accordingly, the pacing electrode 106 can be implanted at the target bundle branch, and the housing 108 can be located away from sensitive structures within the heart 102 chamber.

At operation 1306, optionally, the fixation helix 806 can be moved relative to the helix mount 802 to advance the fixation helix 806 into the interventricular septal wall 104. The transport system can press on the tab 1102 to slide the fixation helix 806 over the helix flange 902 and advance the distal tip of the fixation helix 806. The distal tip can puncture the tissue and engage the septal wall 104. For example, the fixation helix 806 can side stitch the septal wall 104. When the fixation helix 806 is attached to the tissue, the housing 108 can be secured relative to the septal wall 104, away from sensitive heart 102 structures. Accordingly, the target bundle branch can be paced while the housing 108 is optimally placed for stable implant.

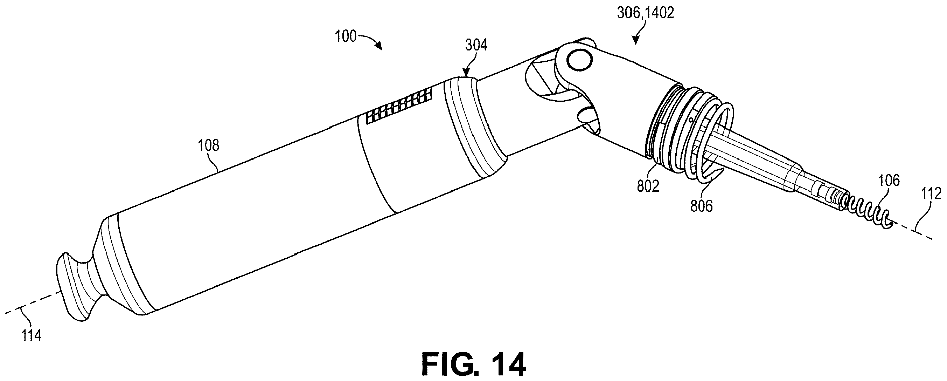

Referring to FIG. 14, a perspective view of a biostimulator having a pivotable electrode is shown in accordance with an embodiment. The biostimulator 100 can be a leadless cardiac pacemaker having components similar to those described above. For example, the biostimulator 100 can include the pacing electrode 106 and the housing 108 containing pacing circuitry. The pacing circuitry can be electrically connected to the pacing electrode 106 via a conductor.

In an embodiment, the joint 306 between the pacing electrode 106 and the housing 108 can include a universal joint 1402. The universal joint 1402 can be a coupling incorporated in the header assembly 304 to interconnect a distal portion of the biostimulator 100 having the pacing electrode 106 to a proximal portion of the biostimulator 100 having the housing 108. The coupling provided by the universal joint 1402 can allow the pacing electrode 106 to pivot relative to the housing 108. For example, the universal joint 1402 can have hinges about which yokes move to allow the electrode axis 112 to incline relative to the housing axis 114. Accordingly, the pacing electrode 106 can be located at the pacing site at a location on the septal wall 104 nearer to the heart valve than the housing 108 while the housing 108 is located, e.g., at the ventricular apex 105 for implant stability.

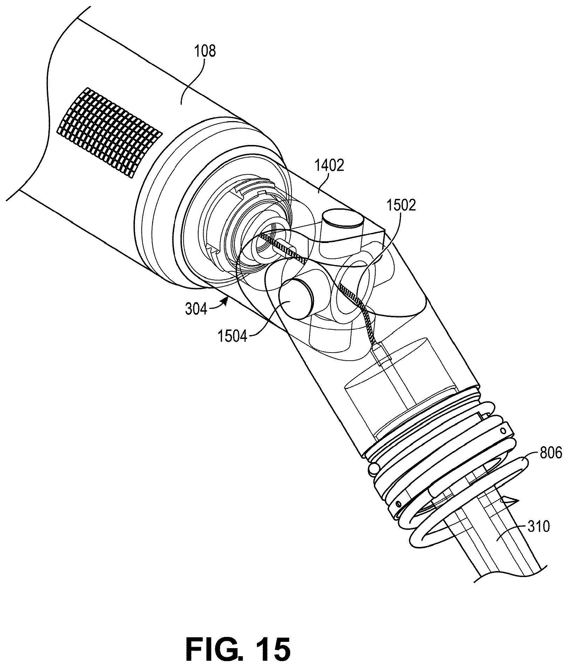

Referring to FIG. 15, a perspective view of a biostimulator having a pivotable electrode is shown in accordance with an embodiment. The biostimulator 100 includes a conductor 1502, e.g., a conducive wire or cable, extending between the pacing circuitry and the pacing electrode 106. For example, the conductor 1502 can have a proximal end electrically connected to the pacing circuitry, e.g., through a feedthrough in the header assembly 304, and a distal end electrically connected to the pacing electrode 106, e.g., through the electrode shaft 310. In an embodiment, the conductor 1502 extends through the universal joint 1402. For example, the conductor 1502 can extend through a spider 1504 of the universal joint 1402. The conductor 1502 may therefore carry pacing signals distally from the pacing circuitry, through the spider 1504 and the universal joint 1402, to the pacing electrode 106.

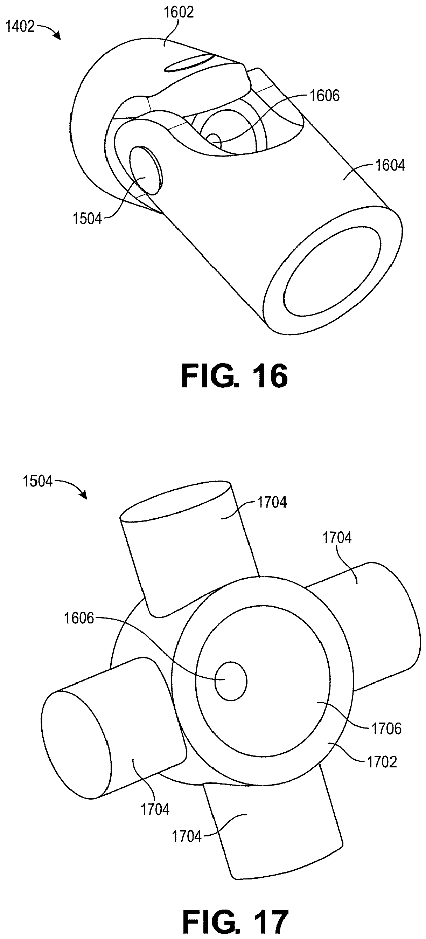

Referring to FIG. 16, a perspective view of a joint of a biostimulator is shown in accordance with an embodiment. The universal joint 1402 may be configured to allow uniform transfer of torque from a proximal portion of the biostimulator 100 to a distal portion of the biostimulator 100 while at different angles. In an embodiment, the universal joint 1402 includes a driving yoke 1602 and a driven yoke 1604 connected to the spider 1504. The driving yoke 1602 can be mechanically connected, e.g., mounted on, the housing 108. For example, the driving yoke 1602 can be a proximal portion of the header assembly 304, which is mounted on the housing 108. The driven yoke 1604 can be can mechanically connected to the pacing electrode 106. For example, the driven yoke 1604 may be a distal portion of the header assembly 304 that supports the helix mount 802, the fixation helix 806, the electrode shaft 310, the pacing electrode 106, etc. The yokes may be mounted on the spider 1504 and, thus, the spider can conjoin the yokes. Torque may be applied to the driving yoke 1602, and the torque may be transmitted through the spider 1504 to the driven yoke 1604. Accordingly, rotation, twisting, or torquing of the housing 108 can be transmitted through the universal joint 1402 to cause corresponding rotation, twisting, or torquing of the pacing electrode 106.

The universal joint 1402 can include a channel 1606 to allow pacing pulses to be delivered centrally through the universal joint 1402. More particularly, the spider 1504 can include the channel 1606, and the conductor 1502 can pass through the channel 1606 of the spider 1504 to conduct electrical signals distally and/or proximally between biostimulator components connected to respective yokes. The channel 1606 can be a through-hole extending through a center of the spider 1504. Accordingly, channel 1606 provides a passage for an electrical pathway to be established along a central axis of the universal joint 1402.

Referring to FIG. 17, a perspective view of a spider of a joint of a biostimulator is shown in accordance with an embodiment. The spider 1504 may be a single, conjoined piece. More particularly, the spider 1504 may include an annulus 1702, having the channel 1606, and several pins 1704 may extend outward from the annulus 1702. The pins 1704 can be integrated with the annulus 1702, e.g., the pins 1704 and the annulus 1702 can be monolithically formed. Accordingly, the spider body can be a rigid component having the centrally-located channel 1606.

A central passage of the universal joint 1402, including the channel 1606, can house the electrical conductor 1502 that connects the pacing circuitry to the pacing electrode 106. More particularly, the conductor 1502 can partially reside within the channel 1606 of the spider 1504. Movement of the universal joint 1402, e.g., pivoting of the driven yoke 1604 with respect to the driving yoke 1602, can cause the conductor 1502 to flex within the channel 1606. In an embodiment, the spider 1504 is formed to reduce fatigue and/or stress on the conductor 1502. The annulus 1702 of the spider 1504 can have a concave funnel feature as a lead-in to the channel 1606. For example, the annulus 1702 can have a tapered surface 1706 extending distally from the channel 1606. The tapered surface 1706, or another funnel feature, can lead into the hole at the center of the spider 1504 from one or both sides of the annulus 1702. In an embodiment, the tapered surface 1706 converges proximally from a distal face of the spider 1504 (shown in FIG. 17) to a longitudinal center of the spider 1504 at which the hole has a minimum dimension, and then expands proximally from the longitudinal center to a proximal face of the spider 1504 (hidden in FIG. 17). The tapered surface 1706 can be rounded, or may otherwise be smooth, to reduce a likelihood of abrading, wearing, or damaging the conductor 1502 when the universal joint 1402 oscillates under in vivo conditions.

The pins 1704 of the spider 1504 can radiate outward from the annulus 1702. For example, the spider 1504 may have four pins 1704, and each pin can extend in a direction orthogonal to adjacent pins 1704. The pins 1704 can form a cross shape relative to the annulus 1702. Accordingly, the driving yoke 1602 and the driven yoke 1604, when connected to respective pairs of diametrically opposed pins, may rotate about axes that are orthogonal to each other. The universal joint 1402 can therefore allow the driven yoke 1604 to pivot orthogonal to the driving yoke 1602, as described below.

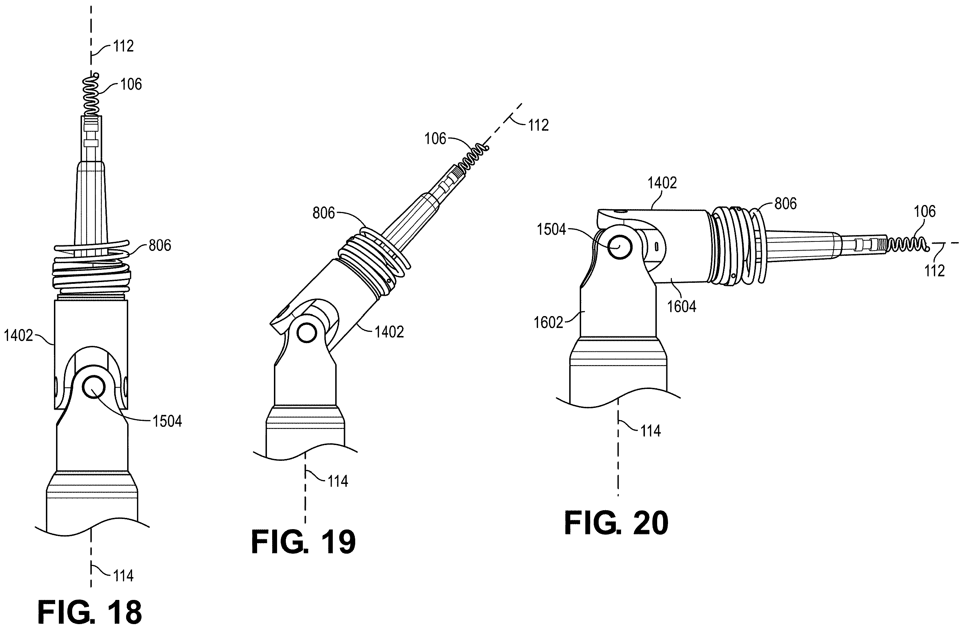

Referring to FIG. 18, a side view of a biostimulator having a pivotable electrode in a pivoted position is shown in accordance with an embodiment. The biostimulator 100 may be in a non-pivoted position. In the non-pivoted position, the electrode axis 112 can be parallel to the housing axis 114. For example, the axes may be co-linear. The universal joint 1402 can be straight, and the spider 1504 may have a transverse plane that is orthogonal to the housing axis 114. The biostimulator 100 may be in the non-pivoted position when contained within a transport system. It will be appreciated, however, that space constraints of the ventricle may not allow the pacing electrode 106 to be inserted normal to the septal wall 104 when the biostimulator 100 is in the non-pivoted position.

Referring to FIG. 19, a side view of a biostimulator having a pivotable electrode in a pivoted position is shown in accordance with an embodiment. The biostimulator 100 may be in an intermediately-pivoted position. In the intermediately-pivoted position, the electrode axis 112 can be oblique to the housing axis 114. For example, the lateral angle 402 between the axes may be between 1 and 89 degrees. It will be appreciated that, in the intermediately-pivoted position, the pacing electrode 106 may be inserted normal to the septal wall 104 while the housing 108 is angled, e.g., upward through the tricuspid valve. The pacing electrode 106 may therefore be engaged and screwed into the septal wall 104 when the biostimulator 100 is in the intermediately-pivoted position.

Referring to FIG. 20, a side view of a biostimulator having a pivotable electrode in a pivoted position is shown in accordance with an embodiment. The biostimulator 100 may be in a fully-pivoted position. In the fully-pivoted position, the electrode axis 112 can be orthogonal to the housing axis 114. Movement of the pacing electrode 106 from the non-pivoted position to the fully-pivoted position is enabled by universal joint 1402. More particularly, the driven yoke 1604 can pivoted about the spider 1504 relative to the driving yoke 1602 from the non-pivoted position to the fully-pivoted position. The universal joint 1402 therefore provides 90° of angular movement freedom between the housing 108 and the pacing electrode 106. It will be appreciated that, in the fully-pivoted position, the housing 108 may be directed downward toward the ventricular apex 105 post-implant. Accordingly, the biostimulator 100 can fit within the space constraints of the ventricle, having the pacing electrode 106 inserted normal to the septal wall 104, and the housing 108 positioned away from sensitive art structures. The biostimulator 100 can therefore deliver therapeutic pacing without interfering with the tricuspid valve or the ventricular free wall.

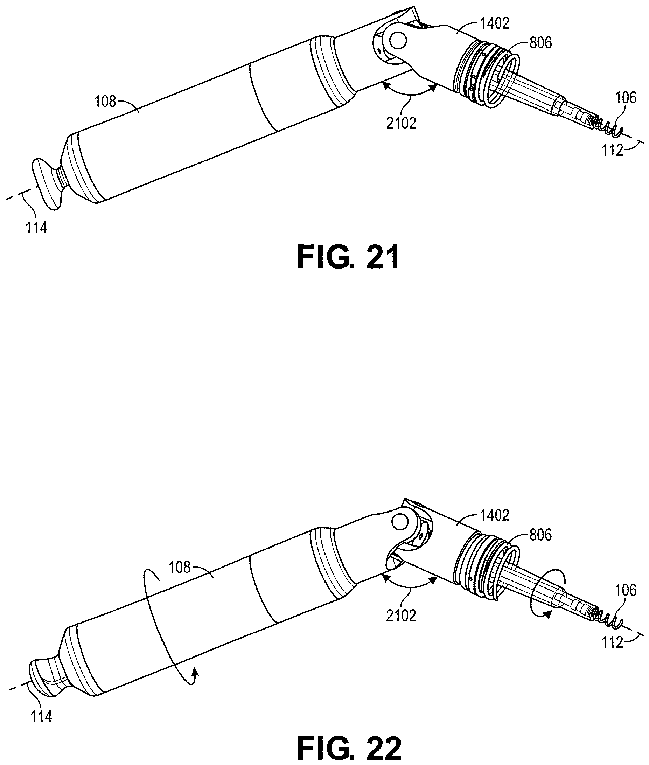

Referring to FIG. 21, a perspective view of a biostimulator having a pivotable electrode in a torqued position is shown in accordance with an embodiment. In addition to allowing the pacing electrode 106 to pivot relative the housing 108, the universal j oint 1402 can allow torque to be efficiently transferred from the housing 108 to the pacing electrode 106 and/or the fixation helix 806. More particularly, the universal joint 1402 can allow torque to be applied when the pacing electrode 106 is at an angle to the housing 108.

The fixation helix 806 can be mounted on the helix mount 802. In an embodiment, the helix mount 802 can be incorporated in a distal portion of the biostimulator 100. For example, the helix mount can be located or mounted on a distal end of the universal joint 1402. Accordingly, rather than affix the housing 108 to the septal wall, as described above, the fixation helix 806 may anchor the distal portion having the pacing electrode 106 to the septal wall. The fixation helix 806 may be movable along the helix mount 802, as described above. Alternatively, the fixation helix 806 may be mounted on and fixed to the helix mount 802. Accordingly, when the universal joint 1402 is rotated, the joint rotation can drive the fixation helix 806 to screw into the septal wall.

When the pacing electrode 106 is inserted normal to the septal wall 104, the electrode axis 112 may be at a driving angle 2102 to the housing axis 114. More particularly, a driving angle 2102 corresponding to an intermediately-pivoted position, e.g., the lateral angle 402, can be between the axes. In the intermediately-pivoted position, e.g., during delivery of the biostimulator 100 to the target site, the pacing electrode 106 and/or the fixation helix 806 can be engaged normal to an upper region of the septal wall 104 and the housing 108 can be angled upward through the tricuspid valve. The biostimulator 100 is therefore pivoted to adapt to the ventricular space.

Referring to FIG. 22, a perspective view of a biostimulator having a pivotable electrode in a torqued position is shown in accordance with an embodiment. The torqued position can be a rotational position of the housing 108 and/or pacing electrode 106 about respective axes. For example, the housing 108 can have a first torqued position when it has a first rotational position and a second torqued position when it is rotated about the housing axis 114 to a second torqued position. More particularly, when the pacing electrode 106 and/or fixation helix 806 are engaged with the upper region of the septal wall 104, torque can be applied to the housing 108 to drive the elements into the septal wall 104. For example, the housing 108 can be torqued to screw the pacing electrode 106 and/or the fixation helix 806 into the interventricular septal wall 104. Notably, the torque can be applied when the distal and proximal portions of the biostimulator 100 are at the driving angle 2102 relative to each other. Furthermore, the driving angle 2102 can be maintained while the torque is applied. More particularly, as the housing 108 and the pacing electrode 106 rotate about respective axes under the applied torque, the universal join pivots such that the electrode axis 112 and the housing axis 114 remain at the driving angle 2102. Maintaining the driving angle 2102 when the pacing electrode 106 screws into the interventricular septal wall 104 allows the housing 108 to remain at a same relative position to the heart. For example, the pacing electrode 106 and/or fixation helix 806 can screw into the target tissue while the housing 108 remains tilted upward through the tricuspid valve. Optimal placement of the pacing electrode 106 can therefore be achieved without interfering with sensitive ventricular structures. The housing 108 may subsequently be released into the region of the ventricular apex 105 to provide long-term pacing and implant stability.

The biostimulator 100 having the universal joint 1402 can include features and/or be used to perform methods described above. For example, the biostimulator 100 illustrated in FIG. 14 can have the fixation helix 806. The fixation helix 806 is described above with respect to FIGS. 9-14 and can be similarly implemented in the embodiment illustrated in FIGS. 14-22. Furthermore, the method illustrated in FIG. 13 may similarly be used to implant the biostimulator 100 for septal pacing. For example, the pacing electrode can be affixed to the septal wall, and the universal joint can be articulated, at operations 1302 and 1304. The method may also include one or more operations to affix the pacing electrode 106 to the septal wall while the universal joint 1402 is pivoted. For example, the method can include an operation at which the housing 108 is torqued to screw the pacing electrode 106 into the septal wall. Accordingly, it will be appreciated that the above descriptions of various embodiments can be combined.

In the foregoing specification, the invention has been described with reference to specific exemplary embodiments thereof. It will be evident that various modifications may be made thereto without departing from the scope of the invention as set forth in the following claims. The specification and drawings are, accordingly, to be regarded in an illustrative sense rather than a restrictive sense.

## Claims

1. A biostimulator (100), comprising:
a pacing electrode (106);
a housing (108) having an electronics compartment (302) containing pacing circuitry electrically connected to the pacing electrode (106); and
a joint (306) between the pacing electrode (106) and the housing (108) such that the pacing electrode (106) is pivotable relative to the housing (108).

2. The biostimulator of claim 1, wherein the pacing electrode (106) includes:
an electrode shaft (310) having a distal shaft end; and
a helical electrode (308) mounted on the distal shaft end.

3. The biostimulator of claim 2, further comprising a masking element (606) covering an outer surface (608) of the electrode shaft (310).

4. The biostimulator of any one of claims 1 to 3, wherein the joint (306) includes a spherical bearing (620).

5. The biostimulator of claim 4, further comprising a header assembly (304) mounted on the housing (108) and having a socket (624), wherein
a ball (622) is connected to the pacing electrode (106); and
the spherical bearing (620) includes the ball (622) in the socket (624).

6. The biostimulator of claim 5, wherein
the header assembly (304) includes a helix mount (802) having a first socket portion (808) and a backplate (810) having a second socket portion (812); and
the backplate (810) is mounted on the helix mount (802) such that the first socket portion (808) and the second socket portion (812) combine to form the socket (624).

7. The biostimulator of any one of claims 1 to 6, further comprising a torque element (330) having a distal element end (332) connected to the pacing electrode (106) and a proximal element end (334) electrically connected to the pacing circuitry.

8. The biostimulator of any one of claims 1 to 7, wherein the pacing electrode (106) includes one or more backstop elements (404).

9. The biostimulator of any one of claims 1 to 8, further comprising a helix mount (802) mounted on the housing (108) and including a fixation helix (806) mounted on the helix mount (802), wherein the fixation helix (806) is movable relative to the helix mount (802).

10. The biostimulator of any one of claims 1 to 9, wherein the joint (306) includes a universal joint (1402).

11. The biostimulator of claim 10, wherein a conductor (1502) extends through the universal joint (1402) between the pacing circuitry and the pacing electrode (106).

12. The biostimulator of claim 11, wherein
the universal joint (1402) includes a driving yoke (1602) and a driven yoke (1604) connected to a spider (1504); and
the conductor (1502) passes through a channel (1606) of the spider (1504).

13. The biostimulator of claim 12, wherein the spider (1504) includes an annulus (1702) having the channel (1606) and a tapered surface (1706) extending distally from the channel (1606).

14. The biostimulator of claim 13, wherein the spider (1504) includes a plurality of pins (1704) radiating outward from the annulus (1702).

15. A biostimulator system (200), comprising:
a biostimulator transport system (202); and
the biostimulator (100) of any of claims 1-14 mounted on the biostimulator transport system (202).
